# DEMANDE DE BREVET EUROPEEN

(11) **EP 1 493 774 A2**
(43) Date de publication de la demande: **05.01.2005**
(21) Numéro de dépôt: 04291413.5
(22) Date de dépôt: 07.06.2004
(51) Int. Cl.: C08G 81/02, A61K 7/00

(54) **Composition pour application topique contenant un polymère hydrosoluble à base de AMPS avec des chaines latérales de polyoxyalkylène**

(30) Priorité: 02.07.2003 FR 0308026
(71) Demandeur: L'OREAL, 75008 Paris (FR)
(72) Inventeur: L'Alloret, Florence, 75013 Paris (FR)
(74) Mandataire: Rasson, Catherine

(57) **Abrégé**

La présente demande concerne une composition aqueuse pour application topique, contenant au moins un polymère hydrosoluble constitué par un squelette hydrosoluble à base d'AMPS et des chaînes latérales comprenant au moins un bloc polyoxyéthylène et au moins un bloc polyoxypropylène ou polyoxybutylène.

Le polymère utilisé présente un effet thermogélifiant et ii a l'avantage d'être peu sensible aux ions multivalents et au pH, et d'avoir une amplitude de l'effet thermogélifiant qui intervient sur une gamme étroite de température, permettant ainsi une transition fluide/gel plus marquée à l'application sur la peau.

## Description

La présente invention concerne une composition aqueuse pour application topique, contenant au moins un polymère hydrosoluble constitué par un squelette hydrosoluble à base d'AMPS et des chaînes latérales comprenant au moins un bloc polyoxyéthylène et au moins un bloc polyoxypropylène et/ou polyoxybutylène.

La plupart des compositions cosmétiques contiennent un ou plusieurs épaississants ou gélifiants utilisés pour ajuster les propriétés de texture en fonction de l'application visée. Les supports galéniques les plus utilisés dans le domaine cosmétique sont d'une part les lotions ou sérums aqueux ne comprenant pas de phase grasse, d'autre part les émulsions qui sont des dispersions de deux liquides immiscibles tels que l'eau et l'huile. Les compositions cosmétiques peuvent également comprendre des particules solides telles que les pigments, les filtres UV inorganiques ou les charges.

Les principaux agents épaississants ou gélifiants hydrophiles utilisés sont les gélifiants polymériques réticulés et les polymères naturels. Comme gélifiants polymériques réticulés, on peut citer par exemple les polymères carboxyvinyliques, comme les produits commercialisés sous les dénominations Carbopol (nom INCl : carbomer) par la société Novéon, les polyacrylamides, les polymères dérivés de l'acide 2-acrylamido-2-méthylpropane sulfonique (AMPS) tels que le produit commercialisé par la société Clariant sous la dénomination Hostacerin AMPS, et les copolymères anioniques réticulés d'acrylamide et d'AMPS, tels que le produit commercialisé sous le nom de SEPIGEL 305 par la société Seppic. Comme polymères naturels, on peut citer par exemple les gommes de xanthane et de guar ou encore les dérivés cellulosiques, les amidons et les alginates. Ces polymères naturels sont en général utilisés en association avec les gélifiants polymériques réticulés car leur pouvoir épaississant est la plupart du temps insuffisamment important pour que l'on puisse les utiliser seuls.

Ces composés voient leur pouvoir gélifiant diminuer lorsque la température augmente, ce qui se traduit par une fluidification des formules à la température de la peau (environ 32°C). Les formules relativement fluides à la température ambiante sont de ce fait difficiles à appliquer sur le visage, et les compositions plus gélifiées deviennent toutes plus fluides à l'application sur la peau.

Par ailleurs, la stabilité des compositions cosmétiques en fonction du temps et sur une large gamme de température (5°C - 50°C) est un paramètre crucial à considérer lors de l'élaboration d'une nouvelle composition. La présence d'une phase dispersée liquide (dans les émulsions) ou solide (dans les suspensions) peut induire des phénomènes de déstabilisation tels que la sédimentation, le crémage, ou encore l'agrégation et la coalescence, cette déstabilisation pouvant se traduire à l'échelle microscopique et/ou macroscopique. Une solution pour limiter ces phénomènes de déstabilisation consiste à augmenter la viscosité de la phase continue, par exemple à l'aide des agents épaississants / gélifiants précédemment cités dans le cas d'une phase continue aqueuse, ce qui implique une concentration élevée en polymère dans la composition qui présente alors un toucher filmogène et collant après application sur la peau.

Il apparaît donc nécessaire de disposer de nouveaux agents gélifiants hydrophiles permettant de diversifier la gamme de textures des compositions cosmétiques aqueuses, notamment avec la possibilité de voir se transformer une composition lors de l'application sur la peau, d'un état fluide à un état gélifié, et aussi d'améliorer la stabilité des compositions au delà de 30°C, spécifiquement pour les compositions peu gélifiées à la température ambiante.

On connaît dans l'état de la technique des polymères particuliers dont la solubilité dans l'eau est modifiée au-delà d'une certaine température. Il s'agit des polymères présentant une température de demixtion par chauffage (ou point de trouble) définissant ainsi leur zone de solubilité dans l'eau. La température de demixtion minimale obtenue en fonction de la concentration en polymère est appelée "LCST" (Lower Critical Solution Temperature).

Ainsi, il est connu, notamment par les documents WO-A-98/29487 et WO-A-97/00275, un système polymérique gélifiant ou épaississant de façon réversible et comprenant une composition soluble dans le milieu considéré, constituée par un composé associatif capable de s'agréger lors d'une augmentation de la température et par un composé soluble dans le milieu considéré et lié au composé associatif. Toutefois, les composés exemplifiés dans le document WO-A-98/29487 présentent l'inconvénient d'être sensibles en présence d'ions multivalents et d'être sensibles au pH. De plus, il n'est pas aisé de contrôler la structure et la nature chimique des polymères décrits dans les documents WO-A-97/00275 et WO-A-98/29487.

Par ailleurs, le document EP-A-1,307,501 se rapporte à des polymères comportant des unités hydrosolubles et des unités à LCST dont la température de démixtion par chauffage à 1% dans l'eau est comprise entre 5°C et 40°C. Les unités à LSCT peuvent être des copolymères statistiques d'oxyéthylène et d'oxypropylène. Toutefois, ces polymères statistiques présentent l'inconvénient d'avoir une grande amplitude de l'effet thermogélifiant qui intervient ainsi sur une large gamme de température.

La présente invention a pour but de pallier les inconvénients de l'art antérieur. La demanderesse a trouvé de manière surprenante des polymères hydrosolubles particuliers permettant de surmonter les inconvénients de ceux décrits dans l'art antérieur. Ces polymères se différencient de ceux de l'art antérieur par le fait qu'ils sont constitués par un squelette hydrosoluble à base d'AMPS et des chaînes latérales comprenant au moins un bloc polyoxyéthylène et au moins un bloc polyoxypropylène ou polyoxybutylène.

Ces polymères présentent l'avantage d'être insensibles en présence d'ions multivalents, d'être insensibles au pH et de donner des compositions ayant de bonnes propriétés cosmétiques. En outre, par rapport aux polymères comportant des copolymères statistiques d'oxyéthylène et d'oxypropylène, ils présentent l'avantage d'avoir une amplitude de l'effet thermogélifiant qui intervient sur une gamme étroite de température, permettant ainsi une transition fluide/gel plus franche à l'application sur la peau et donc une gélification plus importante de la composition appliquée sur la peau.

La demanderesse a constaté que la répartition en blocs des motifs oxyéthylène et oxypropylène/oxybutylène se traduisait par l'existence d'une température critique dite d'agrégation à l'échelle microscopique, c'est-à-dire non visible à l'oeil nu, contrairement aux copolymères statistiques qui présentent dans l'eau, une température inférieure critique de démixtion, au delà de laquelle une séparation macroscopique des phases est observée (point de trouble visible à l'oeil nu). Le phénomène d'agrégation des polymères blocs à l'échelle microscopique correspond à une micellisation pour les polymères triblocs polyoxyéthylène / polyoxypropylène / polyoxyéthylène, comme décrit par Wanka et al. (Colloid and Polymer Science, 1990, (268), 101-117) et Linse et al. (Macromolecules, 1992, (25), 5434-5439), et la température d'agrégation à l'échelle microscopique peut être déterminée par des mesures de microcalorimétrie, comme décrit par Wanka et al. (Colloid and Polymer Science, 1990, (268), 101-117).

La présente invention a donc pour objet une composition pour application topique, notamment cosmétique ou dermatologique, contenant un milieu physiologiquement acceptable et au moins un polymère hydrosoluble constitué par un squelette hydrosoluble à base d'acide 2-acrylamido-2-méthylpropane sulfonique, et par des chaînes latérales comprenant au moins un bloc polyoxyéthylène et au moins un bloc polyoxypropylène et/ou un bloc polyoxybutylène.

La composition selon l'invention étant destinée à une application topique notamment sur la peau, les cheveux ou les phanères, elle contient un milieu physiologiquement acceptable. On entend par "milieu physiologiquement acceptable" un milieu compatible avec les matières kératiniques et notamment la peau, les lèvres, les ongles, le cuir chevelu et/ou les cheveux.

Les polymères utilisés dans la composition de l'invention sont des polymères greffés, dont le squelette hydrosoluble porte des chaînes latérales comprenant au moins un bloc polyoxyéthylène et au moins un bloc polyoxypropylène ou polyoxybutylène. Cette structure peut être partiellement réticulée, par exemple avec un taux de réticulation de 0,01 à 10 % en moles comme indiqué ci-après.

Ces polymères sont susceptibles d'être obtenus à partir de :
(A) de 70 à 99,5 % en mole de motif acide 2-acrylamido-2-méthylpropane sulfonique (AMPS) de formule (I) : 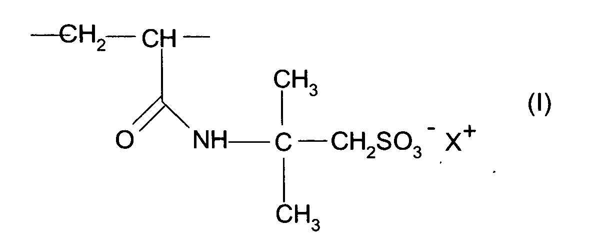 dans laquelle X⁺ est un proton, un cation de métal alcalin, un cation alcalino-terreux ou l'ion ammonium ;
(B) de 0,5% à 30 % en mole, notamment de 2 à 25 % en mole de motif de formule (II) suivante : 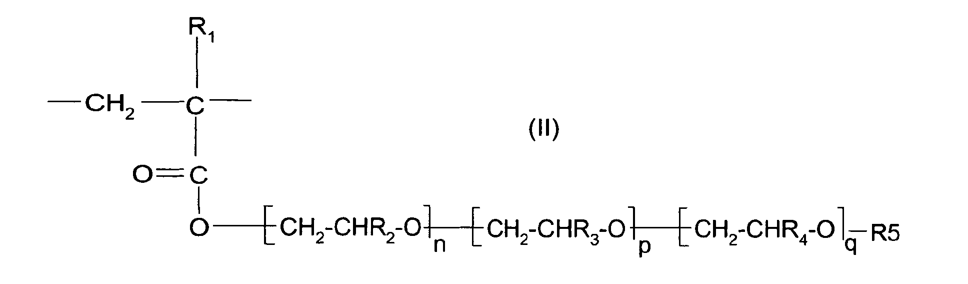 dans laquelle :
   - n, p et q, indépendamment l'un de l'autre, désignent un nombre entier et varient de 0 à 200 ;
   - R₁ désigne un atome d'hydrogène ou un radical alkyle linéaire ou ramifié comportant de 1 à 6 atomes de carbone ;
   - R₂, R₃ et R₄ désignent un atome d'hydrogène ou un radical alkyle comportant de 1 à 2 atomes de carbone, au moins un des radicaux R₂, R₃ ou R₄ étant un atome d'hydrogène, et au moins un des radicaux R₂, R₃ ou R₄ étant un radical alkyle comportant de 1 à 2 atomes de carbone ;
   - R₅ désigne un atome d'hydrogène ou un radical alkyle linéaire, cyclique ou ramifié comportant de 1 à 18 atomes de carbone ;
(C) 0 à 30% en moles de motif hydrosoluble ou hydrophobe portant, avant la polymérisation, au moins une fonction insaturée.

De préférence, la température d'agrégation à l'échelle microscopique des polyéthers de structure blocs de formule (II), à 1 % dans l'eau, va de 5°C à 40°C, et plus spécifiquement de 10°C à 30°C.

De préférence, le motif de formule (II) est choisi parmi ceux où :
- l'un des radicaux R₂ ou R₃ est un atome d'hydrogène et l'autre est un radical alkyle comportant de 1 ou 2 atomes de carbone, et le nombre entier q est égal à 0, n et p étant des nombres entiers entre 0 et 200. Comme copolymères commercialement disponibles correspondant à cette formule, on peut citer par exemple les copolymères diblocs Polyoxyéthylène/Polyoxypropylène alkyl ethers vendus par la société NOF sous les références 502134 (R₅ est un groupe butyle), 523216 (R₅ est un groupe lauryle), 105428, 500442, 109884 (R₅ est un groupe cétyle), 521034 et 522074 (R₅ est un groupe stéaryle) ; ou bien
- R₂ est un atome d'hydrogène, R₃ est un radical méthyle et R₄ et R₅ sont des atomes d'hydrogène, n, p et q étant des nombres entiers entre 0 et 200. Comme copolymères commercialement disponibles correspondant à cette formule, on peut citer par exemple les copolymères triblocs Polyoxyéthylène / Polyoxypropylène / Polyoxyéthylène vendus par la société BASF sous les dénominations de Pluronic, et notamment Pluronic F-127 (n = 106, p = 69 et q = 106), Pluronic P-123 (n = 20, p = 70 et q = 20), Pluronic P-103 (n = 18, p = 58 et q = 18), Pluronic P-104 (n = 25, p = 58 et q = 25), Pluronic P-105 (n = 38, p = 58 et q = 38), Pluronic L-121 (n = 5, p = 69 et q = 5) et Pluronic L-122 (n = 10, p = 69 et q = 10) ; ou bien
- R₂ est un atome d'hydrogène, R₃ est un radical éthyle et R₄ et R₅ sont des atomes d'hydrogène. Comme copolymère commercialement disponible correspondant à cette formule, on peut citer par exemple le copolymère triblocs Polyoxyéthylène / Polybutylène / Polyoxyéthylène vendus par la société NOF sous la dénomination de Plonon B208 (n = 80, p = 28 et q = 80).

Par "motif hydrosoluble" utilisable comme composé (C), on entend des motifs solubles dans l'eau, de 5°C à 80°C, à raison d'au moins 10 g/l, de préférence au moins 20 g/l.

Les motifs hydrophobes, quand ils sont présents comme composé (C), doivent l'être en quantité suffisamment faible pour ne pas entraver la solubilité du squelette du polymère dans l'eau.

Les motifs hydrosolubles sont obtenus à partir d'un ou plusieurs monomères hydrosolubles. Comme monomères hydrosolubles susceptibles d'être employés comme précurseurs des motifs hydrosolubles, seuls ou en mélange, on peut citer à titre d'exemples, les monomères suivants qui peuvent être sous formes d'acide et de sel :
- l'acide (méth)acrylique,
- l'acide styrène sulfonique,
- l'acide vinylsulfonique et l'acide (méth)allylsulfonique,
- le (méth)acrylamide,
- l'acide vinyl phosphonique,
- la N-vinylacétamide et la N-méthyl N-vinylacétamide,
- la N-vinylformamide et la N-méthyl N-vinylformamide,
- les N-vinyllactames comportant un groupe alkyl cyclique ayant de 4 à 9 atomes de carbone, tels que la N-vinylpyrrolidone, la N-butyrolactame et la N-vinylcaprolactame,
- l'anhydride maléique,
- l'acide itaconique,
- l'alcool vinylique de formule CH₂=CHOH,
- les vinyléthers de formule CH₂=CHOR dans laquelle R est un radical hydrocarboné, linéaire ou ramifié, saturé ou insaturé, ayant de 1 à 6 carbones,
- le chlorure de diméthyldiallyl ammonium,
- le méthacrylate de diméthylaminoéthyl quaternisé (MADAME),
- le chlorure de (méth)acrylamidopropyltriméthylammonium (APTAC et MAPTAC),
- le chlorure de méthylvinylimidazolium,
- la 2-vinylpyridine et la 4-vinylpyridine,
- l'acrylonitrile,
- le (méth)acrylate de glycidyle,
- le chlorure de vinyle et le chlorure de vinylidène,
- les monomères vinyliques de formule (A) suivante : 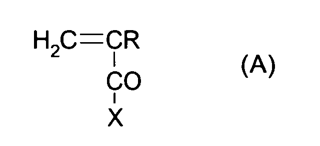
dans laquelle :
- R est choisi parmi H, -CH₃, -C₂H₅ ou -C₃H₇ ;
- X est choisi parmi :
   - les oxydes d'alkyle de type -OR' où R' est un radical hydrocarboné, linéaire ou ramifié, saturé ou insaturé, ayant de 1 à 6 carbones, éventuellement substitué par au moins un atome d'halogène (iode, brome, chlore, fluor); un groupement sulfonique (-SO₃⁻), sulfate (-SO₄⁻), phosphate (-PO₄H₂); hydroxy (-OH); amine primaire (-NH₂); amine secondaire (-NHR₆), tertiaire (-NR₆R₇) ou quaternaire (-N⁺R₆R₇R₈) avec R₆, R₇ et R₈ étant, indépendamment l'un de l'autre, un radical hydrocarboné, linéaire ou ramifié, saturé ou insaturé ayant 1 à 6 atomes de carbone, sous réserve que la somme des atomes de carbone de R' + R₆ + R₇ + R₈ ne dépasse pas 6 ;
   - les groupements -NH₂, -NHR' et -NR'R" dans lesquels R' et R" sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R' + R" ne dépasse pas 6, lesdits R' et R" étant éventuellement substitués par un atome d'halogène (iode, brome, chlore, fluor) ; un groupement hydroxy (-OH); sulfonique (-SO₃⁻); sulfate (-SO₄⁻); phosphate (-PO₄H₂); amine primaire (-NH₂); amine secondaire (-NHR₆), tertiaire (-NR₆R₇) et/ou quaternaire (-N⁺R₆R₇R₈) avec R₆, R₇ et R₈ étant, indépendamment l'un de l'autre, un radical hydrocarboné, linéaire ou ramifié, saturé ou insaturé ayant 1 à 6 atomes de carbone, sous réserve que la somme des atomes de carbone de R' + R" + R₆ + R₇ + R₈ ne dépasse pas 6. Comme composés répondant à cette formule, on peut citer par exemple la N,N-diméthylacrylamide, et la N,N-diéthylacrylamide.
- et leurs mélanges.

Les motifs hydrophobes sont obtenus à partir d'un ou plusieurs monomères hydrophobes. Comme monomères hydrophobes susceptibles d'être employés comme précurseurs des motifs hydrophobes, on peut citer à titre d'exemple, les monomères suivants, seuls ou en mélange, :
- le styrène et ses dérivés tel que le 4-butylstyrène, l'alpha méthylstyrène et le vinyltoluène,
- l'acétate de vinyle de formule CH₂=CH-OCOCH₃ ;
- les vinyléthers de formule CH₂=CHOR dans laquelle R est un radical hydrocarboné, linéaire ou ramifié, saturé ou insaturé, ayant de 1 à 6 carbones;
- l'acrylonitrile,
- la caprolactone,
- le chlorure de vinyle et le chlorure de vinylidène,
- les dérivés siliconés, conduisant après polymérisation à des polymères siliconés tels que le méthacryloxypropyltris(triméthylsiloxy)silane et les méthacrylamides siliconés,
- les monomères vinyliques hydrophobes de formule (B) suivante : 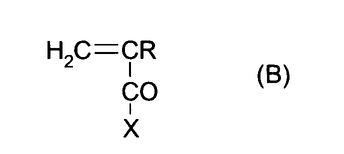
dans laquelle :
- R est choisi parmi H, -CH₃, -C₂H₅ ou -C₃H₇
- X est choisi parmi :
   - les oxydes d'alkyle de type -OR' où R' est un radical hydrocarboné, linéaire ou ramifié, saturé ou insaturé, ayant de 1 à 6 atomes de carbone. Comme composés répondant à cette formule, on peut citer par exemple, le méthacrylate de méthyle, le méthacrylate d'éthyle, le (meth)acrylate de n-butyle, le (meth)acrylate de tertio-butyle, l'acrylate de cyclohexyle et l'acrylate d'isobornyle et l'acrylate d'éthyle 2-hexyle ;
   - les groupements -NH₂, -NHR' et -NR'R" dans lesquels R' et R" sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R' + R" ne dépasse pas 6.
- et leurs mélanges.

Les motifs hydrosolubles ionisables, c'est-à-dire notamment les motifs C et/ou la chaîne AMPS constituant le squelette hydrosoluble, sont, de façon préférentielle, neutralisés partiellement ou totalement par une base minérale (par exemple sodium, ammonium, lithium, calcium, magnésium, ammonium substitué par 1 à 4 groupes alkyles portant de 1 à 15 atomes de carbones) ou une base organique telle que la mono-, di- et tri-éthanolamine, l'aminométhylpropanediol, la N-méthyl-glucamine, les acides aminés basiques comme l'arginine et la lysine, et leurs mélanges.

Le squelette hydrosoluble du polymère utilisé selon l'invention peut être réticulé en utilisant les composés à polyinsaturation oléfinique couramment utilisés comme agents de réticulation pour la réticulation des polymères obtenus par polymérisation radicalaire. On peut citer par exemple comme agents de réticulation, le divinylbenzène, l'éther diallylique, la triallylamine, la tétraallyléthylènediamine, le dipropylèneglycol-diallyléther, les polyglycol-diallyléthers, l'hydroquinone-diallyl-éther, le triméthylolpropane-diallyléther, le tétraallyloxyéthane, les éthers allyliques d'alcools de la série des sucres, les polyallylesters, le tétrallyl-oxéthanoyle ou d'autres allyl- ou vinyléthers alcools polyfonctionnels, le triéthylèneglycol-divinyléther, les esters allyliques de l'acide vinylphosphonique et de l'acide phosphorique, les composés comportant deux ou trois groupes (méth)acrylates ou (méth)acrylamide tels que le diacrylate d'éthylèneglycol, le diacrylate de tétraéthylèneglycol, le diacrylate de butanediol, le méthacrylate d'allyle, le triméthylol propane triacrylate (TMPTA), le méthylène-bis-acrylamide ou leurs mélanges.

Selon un mode préféré de réalisation de l'invention, l'agent de réticulation est de préférence choisi parmi le méthylène-bis-acrylamide, le méthacrylate d'allyle et le triméthylolpropane triacrylate (TMPTA). Le taux de réticulation varie de préférence de 0,01 à 10 % en moles et plus particulièrement de 0,2 à 7 % en moles par rapport au polymère.

De préférence, le squelette hydrosoluble a une masse molaire comprise entre 1000 g/mole et 50 000 000 g/mole, de préférence entre 10 000 g/mole et 10 000 000 g/mole.

Les polymères utilisés selon l'invention sont susceptibles d'être obtenus par les procédés de synthèse classiquement utilisés pour obtenir des polymères «en peigne». On peut citer par exemple les voies de synthèse suivantes :
- Réaction de greffage de chaînes polyéther ayant au moins une extrémité réactive sur des macromolécules hydrosolubles portant des sites réactifs complémentaires, comme par exemple la réaction d'un polyéther ayant une extrémité aminée avec un polymère hydrosoluble portant des fonctions acides carboxyliques, en présence d'un agent de couplage tel que le dicyclohexylcarbodiimide ou le chlorhydrate de 1-(3-diméthylaminopropyl)-3-éthyl carbodiimide. Les polymères obtenus ont une structure «en peigne». Cette voie de synthèse est décrite par exemple par Hourdet (Hourdet D., L'Alloret F., Audebert R., Polymer, 1997, 38 (10), 2535-2547), et dans les documents EP-A-583814, EP-A-629649, WO-A-95/24430 et WO-A-02/09064.
- Copolymérisation d'un macromonomère polyéther et au moins d'un monomère hydrosoluble. Cette voie de synthèse en milieu homogène est décrite par Hourdet (Hourdet D., L'Alloret F., Audebert R., Polymer, 1997, 38 (10), 2535-2547), et dans les documents EP-A-583814, EP-A-629649, WO-A-95/24430 et WO-A-02/09064. Le macromonomère polyéther peut être obtenu par réaction entre un polyéther portant au moins un site réactif et un composé comportant au moins un site réactif complémentaire et au moins une liaison hydrocarbonée insaturée telle qu'une fonction vinyle ou allyle. On peut citer par exemple comme composé de ce type, l'acide (méth)acrylique, l'anhydride maléique, l'acide itaconique, l'alcool vinylique, l'acétate de vinyle, le (méth)acrylate de glycidyle, le 3-chloropropène, le 4-isocyanatostyrène et le chlorométhylstyrène. Un site réactif peut être notamment choisi parmi les fonctions alcool, ester de glycéryle, isocyanate, amine primaire, secondaire ou tertiaire, acide carboxylique ou halogène. Notamment, un site réactif du type acide carboxylique ou ester va généralement réagir avec un site réactif du type alcool ou amine ; un site isocyanate va plutôt réagir avec un site alcool, et un site halogène avec un site alcool ou amine. Les réactions mises en jeu peuvent être par exemple une estérification, une transestérification, une amidification, ou encore une substitution nucléophile.

Le procédé de polymérisation pour la préparation des polymères utilisés dans la composition selon l'invention peut être réalisé en émulsion inverse comme décrit dans le document WO-A-00/35961. Ce procédé de synthèse tel que décrit dans les documents EP-A-1,069,142 et EP-A-1,260,531 est mis en oeuvre dans un milieu hydroalcoolique, conduisant à une polymérisation radicalaire par précipitation. L'alcool utilisé est miscible à l'eau et comporte de 1 à 4 atomes de carbone, comme par exemple le méthanol, l'éthanol, le propanol, l'isopropanol et de préférence le tertiobutanol. Le(s) monomère(s) et le macromonomère sont dissous totalement ou partiellement dans le milieu de polymérisation, tandis que le polymère y est insoluble. La réaction de polymérisation s'effectue à une température comprise entre -10°C et 100°C, de préférence entre 20°C et 70°C. La polymérisation s'effectue en milieu inerte, de préférence sous atmosphère d'azote ou d'argon. Le polymère apparaît dans le milieu réactionnel sous la forme d'un précipité blanc. Il peut être isolé facilement en utilisant les procédés usuels de séparation, d'évaporation et de séchage. Par exemple, l'alcool tel que le tertiobutanol peut être éliminé par filtration ou distillation.

Les polymères utilisés dans la composition selon l'invention sont de préférence présents en une quantité allant de préférence de 0,01 à 20 % en poids, notamment de 0,05 à 15 % en poids, en particulier de 0,1 à 15 % en poids et mieux de 0,1 à 10 % en poids par rapport au poids total de la composition.

Il est possible d'ajouter dans ladite composition selon l'invention, les constituants habituellement utilisés dans le type d'application envisagé. Bien entendu l'homme du métier veillera à choisir ces éventuels constituants complémentaires, et/ou leur quantité, de manière telle que les propriétés avantageuses de la composition selon l'invention ne soient pas, ou substantiellement pas, altérées par l'adjonction envisagée.

La composition selon l'invention est une composition aqueuse. Elle contient donc de l'eau, constituant une phase aqueuse dans laquelle se trouve le polymère utilisé selon l'invention. L'eau y est de préférence présente en une quantité allant de 20 à 99,9 % en poids, en particulier de 40à 95 % en poids par rapport au poids total de la composition.

La composition peut contenir en outre un ou plusieurs composés hydrosolubles et/ou hydrodispersibles, comme des polyols tels que le dipropylène glycol, la glycérine et le butylène glycol, des tensioactifs, des filtres UV hydrosolubles ou hydrodispersibles organiques et minéraux, des actifs, des sels, des charges, des particules organiques, des polymères hydrophiles. Comme polymères hydrophiles, on peut citer par exemple les polymères carboxyvinyliques modifiés ou non, tels que les produits commercialisés sous les dénominations Carbopol (nom INCl : carbomer) et Pemulen (nom INCl : Acrylates/C10-30 akyl acrylate crosspolymer) par la société Novéon ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Clariant sous la dénomination « Hostacerin AMPS » (nom INCl : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom C.T.F.A. : Polyacrylamide / C13-14 Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les polymères neutres synthétiques tels que la poly-N-vinylpyrrolidone ; les polysaccharides comme la gomme de guar, al gomme de xanthane et les dérivés cellulosiques, les dérivés siliconés hydrosolubles ou hydrodispersibles comme les silicones acryliques, les silicones polyéthers et les silicones cationiques.

La composition de l'invention peut comprendre en outre au moins une phase grasse dite aussi phase huileuse, et constituer ainsi des émulsions eau-dans-huile (E/H) ou huile-dans-eau (H/E) ou multiple (E/H/E ou H/E/H), et préférentiellement des émulsions huile-dans-eau (H/E) ou multiple (E/H/E). Quand elle est présente, la phase huileuse peut représenter par exemple de 0,5 à 80 % en poids, de préférence de 1 à 80 % en poids, mieux de 2 à 70 % en poids, encore mieux de 5 à 60 % en poids et préférentiellement de 10 à 40 % en poids par rapport au poids total de la composition.

La phase huileuse contient au moins une huile, plus particulièrement au moins une huile cosmétique. On entend par "huile" un corps gras liquide à la température ambiante (25°C).

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam® ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que le cyclohexadiméthylsiloxane et le cyclopentadiméthylsiloxane ; les polydiméthyl-siloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphényl-méthyldiphényltrisiloxanes, les 2-phényléthyltriméthyl-siloxysilicates, les polyméthylphénylsiloxanes, les huiles siliconées volatiles non cycliques linéaires ou ramifiées ;
- leurs mélanges.

La phase huileuse peut comprendre d'autres corps gras comme par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries. Ces corps gras peuvent être choisis de manière variée par l'homme du métier afin de préparer une composition ayant les propriétés, par exemple de consistance ou de texture, souhaitées.

Quand la composition est une émulsion, elle peut contenir en outre un tensioactif émulsionnant.

Comme exemples de tensioactifs émulsionnants pour les émulsions E/H, on peut citer par exemple les dimethicone copolyols tels que le mélange de cyclomethicone et de dimethicone copolyol, vendu sous la dénomination DC 5225 C par la société Dow Corning ou le diméthicone copolyol vendu sous la dénomination ABIL EM 97 par la société Goldschmidt, et les alkyl-dimethicone copolyols tels que le Laurylmethicone copolyol vendu sous la dénomination Dow Corning 5200 Formulation Aid par la société Dow Corning, le Cetyl dimethicone copolyol vendu sous la dénomination ABIL EM 90 par la société Goldschmidt, ou le mélange polyglycéryl-4 isostéarate /cétyldiméthicone copolyol / hexyllaurate vendu sous la dénomination ABIL WE 09 par la société Goldschmidt. On peut y ajouter aussi un ou plusieurs co-émulsionnants. De manière avantageuse, le coémulsionnant peut être choisi dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Pour les émulsions H/E, on peut citer par exemple comme émulsionnants, les tensioactifs non ioniques, tels que notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, comme les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés ; les éthers d'alcools gras ; les éthers de sucre et d'alcools gras en C₈-C₂₄, et leurs mélanges.

La composition peut contenir en outre tout actif adapté au but recherché. Comme actifs, on peut citer notamment les vitamines (A, C, E, K, PP...) seules ou en mélange, ainsi que leurs dérivés ; les agents kératolytiques et/ou desquamants tels que l'acide salicylique et ses dérivés, les alpha-hydroxyacides, l'acide ascorbique et ses dérivés, les agents anti-inflammatoires, les agents apaisants, les agents dépigmentants, les agents tenseurs tels que les polymères synthétiques, les protéines végétales, les polysaccharides d'origine végétale sous forme ou non de microgels, les amidons, les dispersions de cires, les silicates mixtes et les particules colloïdales de charges inorganiques ; les agents matifiants, les agents anti-chute et/ou repousse des cheveux ou encore les agents anti-rides et leurs mélanges.

La composition selon l'invention peut contenir une ou plusieurs charges minérales ou organiques. Comme charges minérales pouvant être présentes dans la composition selon l'invention, on peut citer par exemple, les pigments minéraux, la poudre de silice, le talc et les particules colloïdales de charges inorganiques telles que les nanoparticules de silices colloïdales comme le produit commercialisé sous la dénomination Cosmo S40 par la société Catalysts and Chemicals et le produit commercialisé sous la dénomination Ludox AM par la société Grace. Comme charges organiques, on peut citer par exemple les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination DRY-FLO par la société National Starch ; les microbilles de résine de silicone telles que celles commercialisées sous la dénomination TOSPEARL par la société Toshiba Silicone ; et leurs mélanges. Quand la composition contient des charges, la quantité de charges peut aller par exemple de 0,05 à 30 % en poids, de préférence de 0,5 à 20 % en poids par rapport au poids total de la composition.

Les filtres U.V. ou agents photoprotecteurs pouvant être utilisés dans la composition conforme à l'invention peuvent être choisis parmi les agents photoprotecteurs organiques et/ou au moins les agents photoprotecteurs inorganiques, actifs dans l'UVA et/ou l'UVB (absorbeurs), hydrosolubles ou liposolubles ou bien insolubles dans les solvants cosmétiques couramment utilisés.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les documents US-A-4367390, EP-A-863145, EP-A-517104, EP-A-570838, EP-A-796851, EP-A-775698, EP-A-878469, EP-A-933376, EP-A-507691, EP-A-507692, EP-A-790243, EP-A-944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les documents EP-A-669323 et US-A-2,463,264 ; les dérivés de l'acide p-aminobenzoïque (PABA) ; les dérivés de méthylène bis-(hydroxyphényl benzotriazole) tels que décrits dans les documents US-A-5,237,071, US-A-5,166,355, GB-A-2303549, DE-A-197 26 184 et EP-A-893119 ; les polymères filtres et silicones filtres tels que ceux décrits notamment dans le document WO-A-93/04665 ; les dimères dérivés d'α-alkylstyrène tels que ceux décrits dans le document DE-A-19855649.

Comme exemples d'agents photoprotecteurs actifs dans l'UV-A et/ou l'UV-B, on peut citer plus particulièrement, ceux désignés ci-dessous sous leur nom INCI :
- les dérivés de l'acide para-aminobenzoïique, dont les suivants : PABA, Ethyl PABA, Ethyl Dihydroxypropyl PABA, Ethylhexyl Diméthyl PABA vendu notamment sous le nom ESCALOL 507 par ISP, Glyceryl PABA, PEG-25 PABA vendu sous le nom UVINUL P25 par BASF,
- les dérivés salicyliques, dont les suivants : Homosalate vendu notamment sous le nom NEO HELIOPAN OS par Haarmann et Reimer, Dipropyleneglycol Salicylate vendu notamment sous le nom DIPSAL par Scher, TEA Salicylate vendu notamment sous le nom NEO HELIOPAN TS par Haarmann et Reimer,
- les dérivés du dibenzoylméthane, dont les suivants : Butyl Methoxydibenzoylmethane vendu notamment sous le nom commercial PARSOL 1789 par Hoffmann Laroche, et Isopropyl dibenzoylmethane,
- les dérivés cinnamiques, dont les suivants : Ethylhexyl Methoxycinnamate vendu notamment sous le nom commercial PARSOL MCX par Hoffmann Laroche, Isopropyl Methoxycinnamate, Isoamyl Methoxycinnamate vendu notamment sous le nom commercial NEO HELIOPAN E 1000 par Haarmann et Reimer, Cinoxate, DEA Methoxycinnamate, Diisopropyl Methylcinnamate, Glyceryl Ethylhexanoate Dimethoxycinnamate,
- les dérivés de β,β-diphénylacrylate, dont les suivants : Octocrylene vendu notamment sous le nom commercial UVINUL N539 par BASF, Etocrylene vendu notamment sous le nom commercial UVINUL N35 par BASF,
- les dérivés de la benzophénone, dont les suivants : Benzophenone-1 vendue notamment sous le nom commercial UVINUL 400 par BASF, Benzophenone-2 vendue notamment sous le nom commercial UVINUL D50 par BASF, Benzophenone-3 ou Oxybenzone vendue notamment sous le nom commercial UVINUL M40 par BASF, Benzophenone-4 vendue notamment sous le nom commercial UVINUL MS40 par BASF, Benzophenone-5, Benzophenone-6 vendue notamment sous le nom commercial HELISORB 11 par Norquay, Benzophenone-8 vendue notamment sous le nom commercial SPECTRA-SORB UV-24 PAR American Cyanamid, Benzophenone-9 vendue notamment sous le nom commercial UVINUL DS-49 par BASF, Benzophenone-12, et le 2-(4-diéthylamino-2-hydroxybenzoyl)-benzoate de n-hexyle ;
- les dérivés du benzylidène camphre, dont les suivants : 3-Benzylidene camphor, 4-Methylbenzylidene camphor vendu notamment sous le nom EUSOLEX 6300 par Merck , Benzylidene Camphor Sulfonic Acid, Camphor Benzalkonium Methosulfate, Terephthalylidene Dicamphor Sulfonic Acid (Mexoryl SX de la société Chimex), Polyacrylamidomethyl Benzylidene Camphor,
- les dérivés de benzimidazole, dont les suivants : Phenylbenzimidazole Sulfonic Acid vendu notamment sous le nom commercial EUSOLEX 232 par Merck, Disodium Phenyl Dibenzimidazole Tetra-sulfonate vendu notamment sous le nom commercial NEO HELIOPAN AP par Haarmann et Reimer,
- les dérivés de triazine, dont les suivants : Anisotriazine vendu notamment sous le nom commercial TINOSORB S par Ciba Specialty Chemicals, Ethylhexyl triazone vendu notamment sous le nom commercial UVINUL T150 par BASF, Diethylhexyl Butamido Triazone vendu notamment sous le nom commercial UVASORB HEB par Sigma 3V, et la 2,4,6- tris-(4'amino-benzalmalonate de diisobutyle)-s-triazine,
- les dérivés de benzotriazole, dont les suivants : Drometrizole Trisiloxane vendu sous le nom SILATRIZOLE par Rhodia Chimie, Methylène bis-Benzotriazolyl Tetramethylbutylphénol, vendu notamment sous forme solide sous le nom commercial MIXXIM BB/100 par Fairmount Chemical ou sous forme micronisée en dispersion aqueuse sous le nom commercial TINOSORB M par Ciba Specialty Chemicals,
- les dérivés anthranilique, dont le Menthyl anthranilate vendu sous le nom commercial NEO HELIOPAN MA par Haarmann et Reimer,
- les dérivés d'imidazolines, dont l'Ethylhexyl Dimethoxybenzylidene Dioxoimidazoline Propionate,
- les dérivés de benzalmalonate, dont le polyorganosiloxane à fonctions benzalmalonate vendu sous la dénomination commerciale PARSOL SLX par Hoffmann Laroche,
- et leurs mélanges.

Les agents photoprotecteurs organiques plus particulièrement préférés sont choisis parmi les composés suivants : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de diisobutyle)-s-triazine, Anisotriazine, Ethylhexyl triazone, Diethylhexyl Butamido Triazone, Methylène bis-Benzotriazolyl Tetramethylbutylphénol, Drometrizole Trisiloxane, et leurs mélanges.

Les agents photoprotecteurs inorganiques peuvent être choisis parmi des pigments ou bien encore des nanopigments (taille moyenne des particules primaires: généralement entre 5 nm et 100 nm, de préférence entre 10 nm et 50 nm) d'oxydes métalliques, enrobés ou non enrobés, comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-518772 et EP-A-518773. On peut citer par exemple le nanotitane hydrophile commercialisé sous la dénomination MIRASUN TIW60 par la société Rhodia, et le nanotitane lipophile commercialisé sous la dénomination MT100T par la société TAYCA ou UV Titan M 160 par la société Kemira.

Les filtres U.V. quand ils sont présents dans les compositions selon l'invention, le sont généralement dans des proportions allant de 0,1 à 20 % en poids par rapport au poids total de la composition, et de préférence allant de 0,2 à 15 % en poids par rapport au poids total de la composition.

Les compositions selon l'invention peuvent se présenter sous forme de gels aqueux ou d'émulsions plus ou moins fluides constituant des laits ou des crèmes. Ces compositions sont préparées selon les méthodes usuelles.

Les compositions de l'invention peuvent être utilisées notamment comme produits de soin, de traitement, de protection, de nettoyage (rincé ou non), de démaquillage, et /ou de maquillage des matières kératiniques (peau, cheveux, cuir chevelu, cils, sourcils, ongles ou muqueuses), et par exemple comme crèmes de protection, de traitement ou de soin pour le visage, pour les mains ou pour le corps, comme laits corporels de protection ou de soin, comme gels ou mousses pour le soin de la peau et/ou des muqueuses (lèvres).

Par ailleurs, les compositions de l'invention contenant des filtres solaires peuvent être ainsi également utilisées comme produits de protection solaire (protection contre le soleil et/ou les U.V. des appareils à bronzer), notamment pour la peau et/ou les cheveux.

Les compositions peuvent être utilisées aussi comme produits pour le maquillage, notamment le maquillage de la peau, des sourcils, des cils et des lèvres, tels que des crèmes pour le visage, des fonds de teint, des mascaras, des rouges à lèvres. De tels produits contiennent généralement des pigments.

Ainsi, l'invention a pour objet l'utilisation cosmétique d'une composition cosmétique telle que définie ci-dessus, comme produit de soin, comme produit de maquillage, comme produit de protection solaire, comme produit de nettoyage et/ou de démaquillage.

Un autre objet de l'invention est un procédé de traitement cosmétique (non-thérapeutique) d'une matière kératinique telle que la peau, le cuir chevelu, les cheveux, les cils, les sourcils, les ongles ou les muqueuses, caractérisé en ce qu'on applique sur la matière kératinique, une composition cosmétique telle que définie ci-dessus.

L'invention est illustrée plus en détail dans les exemples suivants.

### I. Exemples de synthèse des polymères

### Exemple I/1 : Synthèse par greffage d'un polymère hydrosoluble à base d'AMPS et d'un polymère diblocs (oxyéthylène)₆ b (oxypropylène)₄₀ butyl éther

Dans un réacteur de 1 litre muni d'un réfrigérant, on dissout 3,78 grammes d'un copolymère d'acide acrylique (20 % en mole) et d'acide acrylamido-2-méthyl propane sulfonique (80 % en mole) de masse molaire moyenne 500 000 g/mole dans 715 ml d'eau désionisée sous agitation à 20°C pendant 12 heures. Le pH du milieu réactionnel est alors ajusté à 8 à l'aide d'une solution d'hydroxyde de sodium 1M.

Par ailleurs, on dissout 1,60 grammes de copolymère diblocs (oxyéthylène)₆ b (oxypropylène)₄₀ butyl éther mono-aminé dans 100 ml d'eau désionisée sous agitation à 5°C pendant 30 minutes. La solution obtenue est ajoutée goutte à goutte au milieu réactionnel obtenu dans le réacteur, sous vive agitation.

Puis, on dissout 1,84 grammes de chlorhydrate de 1-(3-diméthylaminopropyl)-3-éthyl carbodiimide dans 50 ml d'eau désionisée sous agitation à 20°C pendant 15 minutes. La solution obtenue est ajoutée sous vive agitation, goutte à goutte, au milieu réactionnel précédent qui est alors chauffé à 60°C pendant 6 heures.

Le milieu réactionnel est refroidi à 20°C puis concentré et précipité dans l'acétone. Le polymère sous forme solide est récupéré par filtration et lavé avec un excès d'acétone. La poudre est broyée et séchée sous vide à 35°C.

On obtient 5,5 g d'un copolymère d'acide acrylique (20 % en mole) et d'acide acrylamido-2-méthylpropane sulfonique (80 % en mole) de masse molaire moyenne 500 000 g/mole greffé par 3 % (en mole) de copolymère diblocs (oxyéthylène)₆ b (oxypropylène)₄₀ butyléther (OE)₆(OP)₃₉ (rendement 94%).

### Exemple I/2 : Synthèse par greffage d'un polymère hydrosoluble à base d'AMPS et de polymère triblocs (oxyéthylène)₂₀ b (oxypropylène)₆₉ b (oxyéthylène)₂₀ (Pluronic P123)

Dans un réacteur de 1 litre muni d'un réfrigérant, on dissout 3,78 grammes d'un copolymère d'acide acrylique (20 % en mole) et d'acide acrylamido-2-méthyl propane sulfonique (80 % en mole) de masse molaire moyenne 500 000 g/mole dans 1385 ml d'eau désionisée sous agitation à 20°C pendant 12 heures. Le pH du milieu réactionnel est alors ajusté à 8 à l'aide d'une solution de soude 1M.

On dissout 4,83 grammes de copolymère blocs (oxyéthylène)₂₀ b (oxypropylène)₆₉ b (oxyéthylène)₂₀ (Pluronic P123) préalablement mono-aminé dans 100 ml d'eau désionisée sous agitation à 5°C pendant 30 minutes. La solution obtenue est ajoutée goutte à goutte au milieu réactionnel obtenu dans le réacteur, sous vive agitation.

On dissout 2,41 grammes de chlorhydrate de 1-(3-diméthylaminopropyl)-3-éthyl carbodiimide dans 50 ml d'eau désionisée sous agitation à 20°C pendant 15 minutes. La solution obtenue est ajoutée sous vive agitation, goutte à goutte, au milieu réactionnel précédent qui est alors chauffé à 60°C pendant 6 heures.

Le milieu réactionnel est refroidi à 20°C puis concentré et précipité dans l'acétone. Le polymère sous forme solide est récupéré par filtration et lavé avec un excès d'acétone. La poudre est broyée et séchée sous vide à 35°C.

On obtient 8,5 g d'un copolymère d'acide acrylique (20 % en mole) et d'acide acrylamido-2-méthylpropane sulfonique (80 % en mole) de masse molaire moyenne 500 000 g/mole greffé par 4 % (en mole) de copolymère triblocs (oxyéthylène)₂₀ b (oxypropylène)₆₉ b (oxyéthylène)₂₀ (rendement 94%).

### Exemple I/3 : Synthèse par copolymérisation d'un polymère hydrosoluble à base d'AMPS et de polymère triblocs (oxyéthylène)₁₀₆ b (oxypropylène)₆₉ b (oxyéthylène)₁₀₆ (Pluronic F127)

On introduit 300 grammes de tertio-butanol dans un réacteur de 2 litres muni d'un agitateur, d'un réfrigérant, d'un thermomètre, d'un bain thermostaté, d'une ampoule d'introduction de réactifs et d'une arrivée de gaz afin de contrôler l'atmosphère au dessus du milieu réactionnel. Le milieu réactionnel est placé sous atmosphère d'azote, après un bullage d'azote pendant 30 minutes. 60 grammes d'acide acrylamido-2-méthylpropane sulfonique (AMPS) sont introduits sous agitation et filet d'azote; le pH du milieu réactionnel est alors égal à 1. De l'ammoniaque gazeux est introduit au dessus du milieu réactionnel jusqu'à ce que son pH soit compris entre 7 et 8. puis, on introduit 60 grammes de méthacrylate de copolymère triblocs (oxyéthylène)₁₀₆ b (oxypropylène)₆₉ b (oxyéthylène)₁₀₆, dans le milieu réactionnel qui est alors maintenu sous agitation pendant 1 heure, le pH étant mesuré de façon continue afin de vérifier que sa valeur se situe entre 7 et 8. L'atmosphère au-dessus du milieu réactionnel est de nouveau saturée par de l'azote afin de diminuer la teneur en oxygène dans la phase liquide à une teneur inférieure à 1 ppm. On introduit ensuite un gramme d'azobisisobutyrolnitrile (AIBN) dans le milieu réactionnel sous azote, et on ajuste la température à 60°C. Dès que la température atteint 60°C, la réaction de polymérisation débute. Après une trentaine de minutes, la température est ajustée à la température d'ébullition du tertio-butanol ; le milieu réactionnel est alors maintenu au reflux sous agitation pendant 2 heures.

Le milieu réactionnel devient alors une suspension visqueuse de polymère dans le tertio-butanol, et le polymère est récupéré par simple filtration du tertiobutanol, suivie d'un séchage sous vide.

Le copolymère obtenu est composé de 50 % en masse d'AMPS (sel d'ammonium) et de 50 % de méthacrylate de copolymère triblocs (oxyéthylène)₁₀₆ b (oxypropylène)₆₉ b (oxyéthylène)₁₀₆, ce qui correspond à une proportion molaire de 1,65 % de copolymère triblocs dans le copolymère final.

### Exemple I/4 : Synthèse par copolymérisation d'un polymère hydrosoluble à base d'AMPS et de polymère diblocs (oxyéthylène)₁₀ b (oxypropylène)₃₀ stéarylether

On introduit 300 grammes de tertio-butanol dans un réacteur de 2 litres muni d'un agitateur, d'un réfrigérant, d'un thermomètre, d'un bain thermostaté, d'une ampoule d'introduction de réactifs et d'une arrivée de gaz afin de contrôler l'atmosphère au-dessus du milieu réactionnel. Le milieu réactionnel est placé sous atmosphère d'azote, après un bullage d'azote pendant 30 minutes. 60 grammes d'acide acrylamido-2-méthylpropane sulfonique (AMPS) sont introduits sous agitation et filet d'azote, le pH du milieu réactionnel étant alors égal à 1. De l'ammoniaque gazeux est introduit au-dessus du milieu réactionnel jusqu'à ce que son pH soit compris entre 7 et 8. Puis, on introduit 60 grammes de polymère diblocs (oxyéthylène)₁₀ b (oxypropylène)₃₀ stéarylether dans le milieu réactionnel qui est alors maintenu sous agitation pendant 1 heure, le pH étant mesuré de façon continue afin de vérifier que sa valeur se situe entre 7 et 8. L'atmosphère au-dessus du milieu réactionnel est de nouveau saturée par de l'azote afin de diminuer la teneur en oxygène dans la phase liquide à une teneur inférieure à 1 ppm. Ensuite, on introduit un gramme d'azobisisobutyrolnitrile (AIBN) dans le milieu réactionnel sous azote et on ajuste la température à 60°C. Dès que la température atteint 60°C, la réaction de polymérisation débute. Après une trentaine de minutes, la température est ajustée à la température d'ébullition du tertio-butanol; le milieu réactionnel est alors maintenu au reflux sous agitation pendant 2 heures.

Le milieu réactionnel devient alors une suspension visqueuse de polymère dans le tertio-butanol, et le polymère est récupéré par simple filtration du tertiobutanol, suivie d'un séchage sous vide.

Le copolymère obtenu est composé de 50 % en masse d'AMPS (sel d'ammonium) et de 50 % de méthacrylate de copolymère diblocs (oxyéthylène)₁₀ b (oxypropylène)₃₀ stéarylether, ce qui correspond à une proportion molaire de 8,5 % de copolymère diblocs dans le copolymère final.

### II. Exemples de composition

Les quantités indiquées sont en % en poids sauf mention contraire.

### Exemple II/1 selon l'invention : Gel hydratant comprenant le polymère de l'exemple I/1

| | |
|---|---|
| Polymère de l'exemple I/1 | 2 g |
| Glycérine | 3 g |
| Conservateur | 0,2 g |
| Eau déminéralisée | 94,8 g |

Le pouvoir thermogélifiant de cette composition a été mis en évidence par des mesures rhéologiques (Rhéomètre Haake RS150, cône/plan 35mm/2°), à une vitesse de cisaillement de 10 s⁻¹ :
- Viscosité à 20°C : 0,02 Pa.s
- Viscosité à 32°C : 50 Pa.s
- Température de gélification : 25 °C

### Exemple II/2 comparatif : Gel hydratant comprenant un copolymère d'acide acrylique (20 % en mole) et d'AMPS (80 % en mole) portant 3 % (en mole) de chaînes latérales copolymère statistique (oxyéthylène)₆ (oxypropylène)₄₀ butyl éther

| | |
|---|---|
| Copolymère d'acide acrylique (20 % en mole) et d'AMPS (80% en mole) portant 3 % (en mole) de chaînes latérales copolymère statistique (oxyéthylène)₆ (oxypropylène)₄₀ butyl éther | 2 g |
| Glycérine | 3 g |
| Conservateur | 0,2 g |
| Eau déminéralisée | 94,8 g |

Le pouvoir thermogélifiant de cette composition a été mis en évidence par des mesures rhéologiques (Rhéomètre Haake RS150, cône/plan 35mm/2°), à une vitesse de cisaillement de 10 s⁻¹.
- Viscosité à 20°C : 0,015 Pa.s
- Viscosité à 32°C : 1 Pa.s
- Température de gélification : 28 °C

Cette composition présente une température de gélification proche de celle de la composition de l'exemple II/1 selon l'invention, mais l'amplitude de l'effet thermogélifiant entre 20°C et 32°C est beaucoup moins importante. La transition fluide/gel à l'application est moins prononcée et conduit à des effets sensoriels moins marqués.

### Exemple II/3 selon l'invention : Lait pour le corps

| Phase aqueuse : | |
|---|---|
| Polymère de l'exemple I/2 | 0,4 g |
| Conservateur | 0,2 g |
| Eau déminéralisée | 84,4 g |

| Phase huileuse : | |
|---|---|
| Huile de Parléam | 9 g |
| Cyclohexadiméthylsiloxane | 6 g |

La phase aqueuse est préparée par dissolution du polymère de l'exemple I/2 à la température ambiante dans de l'eau déminéralisée contenant le conservateur, sous agitation pendant 2 heures. La phase huileuse est alors introduite lentement dans la phase aqueuse sous agitation à l'aide d'un mélangeur de type Moritz avec une vitesse de 4000 RPM pendant 20 minutes.

Le polymère de l'exemple I/2 permet à lui seul d'émulsionner la totalité de la phase huileuse. La composition obtenue est une belle émulsion fluide pouvant être utilisée comme lait pour le corps.

## Revendications

1. Composition aqueuse pour application topique, contenant un milieu physiologiquement acceptable et au moins un polymère hydrosoluble constitué par un squelette hydrosoluble à base d'acide 2-acrylamido-2-méthylpropane sulfonique, et par des chaînes latérales comprenant au moins un bloc polyoxyéthylène et au moins un bloc polyoxypropylène et/ou un bloc polyoxybutylène.

2. Composition selon la revendication 1, **caractérisée en ce que** le polymère est susceptible d'être obtenu à partir de :
(A) de 70 à 99,5 % en mole de motif acide 2-acrylamido 2-méthylpropane sulfonique de formule (I) : 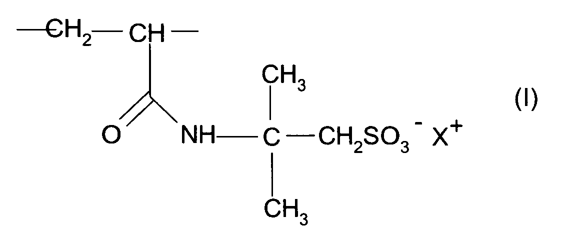 dans laquelle X⁺ est un proton, un cation de métal alcalin, un cation alcalino-terreux ou l'ion ammonium ;
(B) de 0,5% à 30 % en mole de motif de formule (II) suivante : 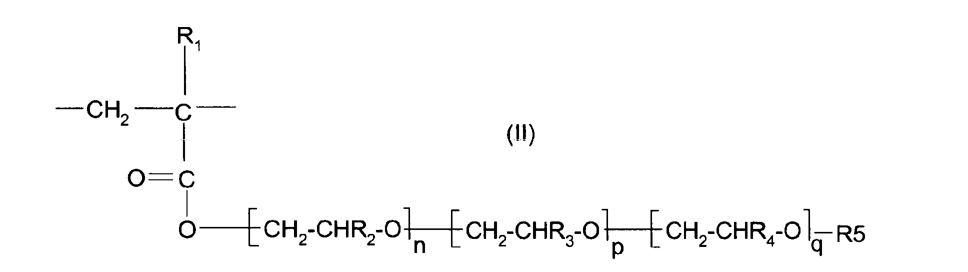 dans laquelle :
- n, p et q, indépendamment l'un de l'autre, désignent un nombre entier et varient de 0 à 200 ;
- R₁ désigne un atome d'hydrogène ou un radical alkyle linéaire ou ramifié comportant de 1 à 6 atomes de carbone ;
- R₂, R₃ et R₄ désignent un atome d'hydrogène ou un radical alkyle comportant de 1 à 2 atomes de carbone, au moins un des radicaux R₂, R₃ ou R₄ étant un atome d'hydrogène, et au moins un des radicaux R₂, R₃ ou R₄ étant un radical alkyle comportant de 1 à 2 atomes de carbone ;
- R₅ désigne un atome d'hydrogène ou un radical alkyle linéaire, cyclique ou ramifié comportant de 1 à 18 atomes de carbone, et
(C) 0 à 30% en moles de motif hydrosoluble ou hydrophobe portant, avant la polymérisation, au moins une fonction insaturée.

3. Composition selon la revendication précédente, **caractérisé en ce que** le motif de formule (II) est choisi parmi ceux où :
- l'un des radicaux R₂ ou R₃ est un atome d'hydrogène et l'autre est un radical alkyle comportant de 1 à 2 atomes de carbone, et le nombre entier q est égal à 0 ; ou bien
- R₂ est un atome d'hydrogène, R₃ est un radical méthyle et R₄ et R₅ sont des atomes d'hydrogène ; ou bien
- R₂ est un atome d'hydrogène, R₃ est un radical éthyle et R₄ et R₅ sont des atomes d'hydrogène.

4. Composition selon la revendication 2 ou 3, **caractérisée en ce que** le motif hydrosoluble (C) est obtenu à partir d'un ou plusieurs monomères hydrosolubles choisis parmi les monomères suivants qui peuvent être sous formes d'acide et de sel :
- l'acide (méth)acrylique,
- l'acide styrène sulfonique,
- l'acide vinylsulfonique et l'acide (méth)allylsulfonique,
- le (méth)acrylamide,
- l'acide vinyl phosphonique,
- la N-vinylacétamide et la N-méthyl N-vinylacétamide,
- la N-vinylformamide et la N-méthyl N-vinylformamide,
- les N-vinyllactames comportant un groupe alkyl cyclique ayant de 4 à 9 atomes de carbone,
- l'anhydride maléique,
- l'acide itaconique,
- l'alcool vinylique,
- les vinyléthers de formule CH₂=CHOR dans laquelle R est un radical hydrocarboné, linéaire ou ramifié, saturé ou insaturé, ayant de 1 à 6 carbones,
- le chlorure de diméthyldiallylammonium,
- le méthacrylate de diméthylaminoéthyl quaternisé,
- le chlorure de (méth)acrylamidopropyltriméthylammonium,
- le chlorure de méthylvinylimidazolium,
- la 2-vinylpyridine et la 4-vinylpyridine,
- l'acrylonitrile,
- le (méth)acrylate de glycidyle,
- le chlorure de vinyle et le chlorure de vinylidène,
- les monomères vinyliques de formule (A) suivante : 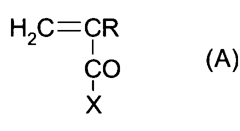
dans laquelle :
- R est choisi parmi H, -CH₃, -C₂H₅ ou -C₃H₇
- X est choisi parmi :
- les oxydes d'alkyle de type -OR' où R' est un radical hydrocarboné, linéaire ou ramifié, saturé ou insaturé, ayant de 1 à 6 carbones, éventuellement substitué par au moins un atome d'halogène (iode, brome, chlore, fluor); un groupement sulfonique (-SO₃⁻), sulfate (-SO₄⁻), phosphate (-PO₄H₂); hydroxy (-OH); amine primaire (-NH₂); amine secondaire (-NHR₆), tertiaire (-NR₆R₇) ou quaternaire (-N⁺R₆R₇R₈) avec R₆, R₇ et R₈ étant, indépendamment l'un de l'autre, un radical hydrocarboné, linéaire ou ramifié, saturé ou insaturé ayant 1 à 6 atomes de carbone, sous réserve que la somme des atomes de carbone de R' + R₆ + R₇ + R₈ ne dépasse pas 6 ;
- les groupements -NH₂, -NHR' et -NR'R" dans lesquels R' et R" sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R' + R" ne dépasse pas 6, lesdits R' et R" étant éventuellement substitués par un atome d'halogène (iode, brome, chlore, fluor) ; un groupement hydroxy (-OH); sulfonique (-SO₃⁻); sulfate (-SO₄⁻); phosphate (-PO₄H₂); amine primaire (-NH₂); amine secondaire (-NHR₆), tertiaire (-NR₆R₇) et/ou quaternaire (-N⁺R₆R₇R₈) avec R₆, R₇ et R₈ étant, indépendamment l'un de l'autre, un radical hydrocarboné, linéaire ou ramifié, saturé ou insaturé ayant 1 à 6 atomes de carbone, sous réserve que la somme des atomes de carbone de R' + R" + R₆ + R₇ + R₈ ne dépasse pas 6 ;
- et leurs mélanges.

5. Composition selon l'une quelconque des revendications 2 à 4, **caractérisée en ce que** le motif hydrophobe (C) est obtenu à partir d'un ou plusieurs monomères hydrophobes choisis parmi les monomères suivants :
- le styrène et ses dérivés,
- l'acétate de vinyle,
- les vinyléthers de formule CH₂=CHOR dans laquelle R est un radical hydrocarboné, linéaire ou ramifié, saturé ou insaturé, ayant de 1 à 6 carbones;
- l'acrylonitrile,
- la caprolactone,
- le chlorure de vinyle et le chlorure de vinylidène,
- les dérivés siliconés, conduisant après polymérisation à des polymères siliconés,
- les monomères vinyliques hydrophobes de formule (B) suivante : 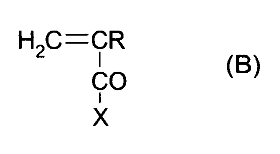
dans laquelle :
- R est choisi parmi H, -CH₃, -C₂H₅ ou -C₃H₇
- X est choisi parmi:
- les oxydes d'alkyle de type -OR' où R' est un radical hydrocarboné, linéaire ou ramifié, saturé ou insaturé, ayant de 1 à 6 atomes de carbone ;
- les groupements -NH₂, -NHR' et -NR'R" dans lesquels R' et R" sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R' + R" ne dépasse pas 6 ;
- et leurs mélanges.

6. Composition selon l'une quelconque des revendications précédentes, **caractérisée en ce que** le squelette hydrosoluble et/ou le motif hydrosoluble (C) sont neutralisés partiellement ou totalement par une base minérale ou une base organique.

7. Composition selon l'une quelconque des revendications précédentes, **caractérisée en ce que** le squelette hydrosoluble est réticulé par un agent de réticulation choisi parmi le méthylène-bis-acrylamide, le méthacrylate d'allyle et le triméthylolpropane triacrylate.

8. Composition selon l'une des revendications précédentes, **caractérisée en ce que** le squelette hydrosoluble a une masse molaire comprise entre 1000 g/mole et 50 000 000 g/mole, de préférence entre 10 000 g/mole et 10 000 000 g/mole.

9. Composition selon l'une des revendications précédentes, **caractérisée en ce que** le polymère est susceptible d'être obtenu par réaction de greffage de chaînes polyéther ayant au moins une extrémité réactive sur des macromolécules hydrosolubles portant des sites réactifs complémentaires, ou par copolymérisation d'un macromonomère polyéther et au moins d'un monomère hydrosoluble.

10. Composition selon l'une des revendications précédentes, **caractérisée en ce que** le polymère est présent en une quantité allant de 0,01 à 20 % en poids, et de préférence de 0,05 à 15 % en poids par rapport au poids total de la composition.

11. Composition selon l'une quelconque des revendications précédentes, **caractérisée en ce qu'**elle comporte une quantité d'eau allant de 20 à 99,9 % en poids, par rapport au poids total de la composition.

12. Composition selon l'une quelconque des revendications précédentes, **caractérisée en ce qu'**elle comporte une phase huileuse.

13. Composition selon l'une quelconque des revendications précédentes, **caractérisée en ce qu'**elle constitue une composition cosmétique ou dermatologique.

14. Composition selon l'une quelconque des revendications 1 à 12, **caractérisée en ce qu'**elle constitue un produit de soin, de traitement, de protection, de nettoyage, de démaquillage et /ou de maquillage des matières kératiniques, notamment la peau.

15. Utilisation cosmétique d'une composition cosmétique selon l'une quelconque des revendications 1 à 12, comme produit de soin, comme produit de maquillage, comme produit de protection solaire, ou comme produit de nettoyage et/ou de démaquillage.

16. Procédé de traitement cosmétique d'une matière kératinique, **caractérisé en ce que** l'on applique sur la matière kératinique, une composition cosmétique telle que définie selon l'une quelconque des revendications 1 à 12.
